# EUROPÄISCHE PATENTSCHRIFT

(11) **EP 0 627 201 B1**
(45) Veröffentlichungstag und Bekanntmachung des Hinweises auf die Patenterteilung: **22.07.1998**
(21) Anmeldenummer: 93810398.3
(22) Anmeldetag: 02.06.1993
(51) Int. Cl.: A61F 2/06

(54) **Vorrichtung zum Freisetzen einer selbstexpandierenden Endoprothese**
Device for releasing a self-expanding endoprosthesis
Dispositif pour libérer une endoprothèse auto-expansible

(43) Veröffentlichungstag der Anmeldung: 07.12.1994
(73) Patentinhaber: Schneider (Europe) GmbH, 8180 Bülach (CH)
(72) Erfinder: Lukic, Goran, CH-8180 Bülach (CH); Hofmann, Eugen, CH-8064 Zürich (CH)
(74) Vertreter: Groner, Manfred

(56) Entgegenhaltungen:
- EP-A- 350 043
- US-A- 4 665 918
- US-A- 4 732 152
- US-A- 5 201 757

## Beschreibung

Die Erfindung betrifft eine Vorrichtung zum Freisetzen einer selbstexpandierenden Endoprothese, mit einem flexiblen länglichen Aussenkatheter mit einem distalen Ende und einem proximalen Ende und mit einem koaxial zum Aussenkatheter angeordneten flexiblen länglichen Innenkatheter, der an einem distalen Ende eine Spitze und proximal dieser Mittel zur Aufnahme der Endoprothese aufweist, wobei zur Fixierung der Endoprothese der Aussenkatheter in seiner Längsrichtung über die Mittel schiebbar und zu deren Freigabe zurückziehbar ist.

Eine Vorrichtung dieser Gattung sowie Endoprothesen sind beispielsweise durch die US-A-5,026,377 bekannt geworden. Mit dieser Vorrichtung kann eine Endoprothese, auch Stent oder Gefässstütze genannt, implantiert werden. Eine solche Implantation erfolgt beispielsweise nach einer Ballondilatation einer Stenose, um eine Rezitivstenose zu verhüten. Sie können jedoch auch beispielsweise in Harn-, Gallen- oder Venenwege implantiert werden, um einen Verschluss dieser Wege zu verhindern. Die Endoprothese besteht beispielsweise aus einem rohrförmigen Gewebe aus rostfreien Stahldrähten. Mit der genannten Vorrichtung wird eine Endoprothese in gespanntem Zustand an die vorgesehene Stelle gebracht und durch einen Rückzug des schlauchförmigen Aussenkatheters freigesetzt. Hierbei dehnt sich die Endoprothese selbsttätig auf Kosten ihrer Länge radial aus und spannt sich gegen die Gefässwand.

Eine Vorrichtung dieser Art ist auch aus der US-A-4,665,918 bekannt geworden. Diese wird wie üblich durch Einführungskatheter (guiding catheter) in ein Gefäss so weit eingeführt, bis das distale Ende über dasjenige des Einführungskatheters hinausragt und der Stent an die vorgesehene Stelle gebracht ist. Anschliessend wird auch hier der Stent durch einen Rückzug eines schlauchförmigen Aussenkatheters freigesetzt.

Aus der US-A-5201757 ist eine ähnliche Vorrichtung mit insgesamt drei Kathetern bekannt. Dabei ist der innerste Katheter mit einer Spitze und einem sich von der Spitze proximal erstreckenden Rohrstück fest verbunden. Der mittlere Katheter trägt Mittel zur Aufnahme der Endoprothese, die dann teils vom genannten Rohrstück, teils vom äußeren Katheter bis zu ihrer Freisetzung radial komprimiert gehalten wird.

Die genannte Vorrichtung hat sich in der Praxis an sich bewährt. Jedoch sind mit dieser solche Endoprothesen schwierig freizusetzen, die aussen mit einer Beschichtung versehen sind und die als sogenannte "covered stents" bekannt sind und gewisse wesentliche Vorteile besitzen. Ebenfalls verursacht die Freisetzung sehr langer Endoprothesen und solcher mit grosser Expansionsspannung Schwierigkeiten. Schliesslich sind auch Endoprothesen nur schwierig freizusetzen, die Drähte mit einer porösen Oberfläche besitzen. Die genannten Schwierigkeiten beim Freisetzen solcher Endoprothesen bestehen insbesondere darin, dass der Aussenkatheter nicht oder nur mit vergleichsweise hoher Kraft zurückgezogen werden kann.

Der Erfindung liegt die Aufgabe zugrunde, eine Vorrichtung der genannten Gattung zu schaffen, welche die oben genannten Schwierigkeiten vermeidet und die dennoch einfach und sicher bedienbar ist.

Die Aufgabe ist bei einer gattungsgemässen Vorrichtung gemäss Kennzeichen des Anspruchs 1 gelöst. Bei der erfindungsgemässen Vorrichtung wird zur Freigabe der Endoprothese zuerst der zweite Aussenkatheter und anschliessend der erste Aussenkatheter zurückgezogen. Da nun die Endoprothese nur über einen Teilbereich am zweiten Aussenkatheter anliegt, muss beim Zurückziehen des zweiten Aussenkatheters lediglich die Reibung dieses Teilbereiches der Endoprothese überwunden werden. Beim Zurückziehen des ersten Aussenkatheters muss dann schliesslich lediglich noch die Reibung des anderen Teilbereiches der Endoprothese überwunden werden. Die Gesamtreibung der Aussenseite der Endoprothese wird somit auf die beiden Aussenkatheter verteilt. Da die Festigkeitsanforderungen an den zweiten Aussenkatheter vergleichsweise klein sind, genügt eine geringe Wandstärke, so dass die Durchmesservergrösserung der Vorrichtung durch den zweiten Aussenkatheter entsprechend gering ist. Ausser der Lösung der gestellten Aufgabe weist die erfindungsgemässe Vorrichtung den wesentlichen Vorteil auf, dass die Endoprothese nach dem Zurückziehen des zweiten Aussenkatheters im ersten Aussenkatheter gehalten ist und die Endoprothese durch vorschieben des zweiten Aussenkatheters wieder zurückgefaltet und somit die Endoprothese neu positioniert oder vollständig wieder aus dem Gefäss entfernt werden kann. Die erfindungsgemässe Vorrichtung bildet deshalb auch beim Implantieren von üblichen Endoprothesen einen wesentlichen Vorteil. Zudem hat sich das Montieren der Endoprothese als besonders einfach erwiesen. Weitere vorteilhafte Merkmale ergeben sich aus den abhängigen Ansprüchen, der nachfolgenden Beschreibung sowie der Zeichnung.

Ein Ausführungsbeispiel der erfindungsgemässen Vorrichtung wird nachfolgend anhand der Zeichnung näher erläutert. Es zeigen:
- Fig. 1: einen Längsschnitt durch das distale Ende einer erfindungsgemässen Vorrichtung, wobei eine Endoprothese in die Vorrichtung aufgenommen ist,
- Fig. 2: das distale Ende gemäss Fig. 1, wobei dieses jedoch in einen Gefässabschnitt eingesetzt und die Endoprothese teilweise freigesetzt ist,
- Fig. 3: einen Schnitt gemäss Fig. 2, wobei jedoch die Endoprothese vollständig freigesetzt ist, und
- Fig. 4: eine Ansicht des proximalen Endes der Vorrichtung.

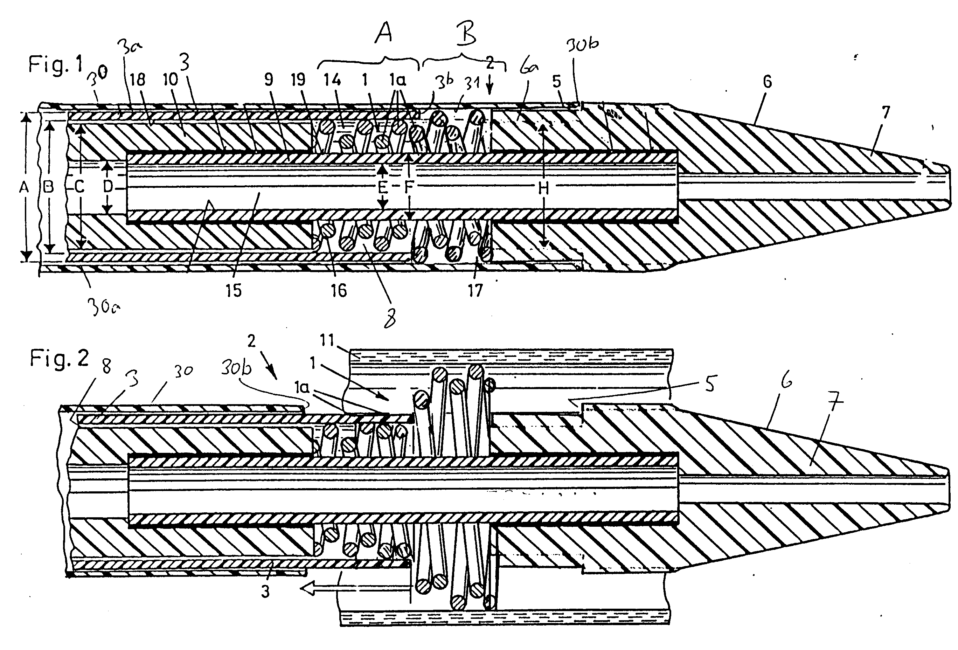

Die Fig. 1 zeigt eine erfindungsgemässe Vorrichtung 2 in der eine Endoprothese 1 montiert ist. Die Endoprothese 1 ist beispielsweise aus rostfreien Stahldrähten 1a geflochten und kann aussenseitig mit einer hier nicht gezeigten dehnbaren Hülle versehen sein. Die Länge der Endoprothese 1 ist selbstverständlich auf die vorgesehene Verwendung abgestimmt und diese Länge kann insbesondere wesentlich grösser als hier gezeigt sein. Die Endoprothese 1 ist in einem proximalen Bereich A über den ganzen Umfang von einem distalen Bereich eines ersten Aussenkatheters 3 und in einem distalen Bereich B von einem zweiten Aussenkatheter 30 überspannt. Die beiden Aussenkatheter 3 und 30 weisen jeweils ein flexibles länglichen Schlauchstück 3a bzw. 30a auf, die jeweils mit einer distalen Mündung 3b bzw. 30b versehen sind. Die Endoprothese 1 liegt somit mit ihrer Aussenseite in einem Teilbereich A unter Spannung an einer Innenseite 18 des ersten Aussenkatheters 3 und in einem Teilbereich B ebenfalls unter Spannung an einer Innenseite 31 des zweiten Aussenkatheters 30 an. Beide Aussenkatheter 3 und 30 verhindern eine radiale Expansion der Endoprothese 1.

Ein flexibler Innenkatheter 6 ist in ein durchgehendes Lumen 8 des ersten Aussenkatheters 3 eingesetzt und dieser Innenkatheter 6 kann ein durchgehendes Lumen 15 zur Aufnahme eines hier nicht gezeigten Führungsdrahtes aufweisen. Der Innenkatheter 6 weist eine vorzugsweise flexible Spitze 7, ein Zwischenstück 9 sowie ein proximal zum Zwischenstück 9 angeordnetes Rohrstück 10 auf. Das flexible Zwischenstück 9 ist aussenseitig zylindrisch und weist einen Aussendurchmesser F auf, der kleiner ist als der Aussendurchmesser C des Rohrstückes 10 und auch kleiner als der Aussendurchmesser H der Spitze 7. Im Abstand zueinander angeordnete Querschnittsflächen 16 und 17 des Rohrstücks 10 und der Spitze 7 sowie die Innenseiten 18 und 31 der Aussenkatheter 3 bzw. 30 sowie eine zylindrische Aussenseite 19 des Zwischenstücks 9 bilden einen hohlzylindrischen Raum 14, in dem die montierte Endoprothese 1 in gespanntem Zustand untergebracht ist. Im Raum 14 ist die Endoprothese 1 in Längsrichtung durch die Flächen 16 und 17 und durch Reibung an den beiden Aussenkathetern 3 und 30 fixiert. Anstelle des Raumes 14 ist es jedoch in bekannter Weise auch möglich, die Endoprothese 1 mit anderen Mitteln axial am Innenkatheter 6 zu fixieren.

Die Spitze 7 greift mit einem Ansatz 6a in das distale Ende des zweiten Aussenkatheters 30 ein und liegt mit einer Schulter 5 an der Mündung 30b dieses Katheters an. Wie ersichtlich übergreift die Schulter 5 die Mündung 30b des Aussenkatheters. Damit ist verhindert, dass beim Einführen der Vorrichtung in ein Gefäss 11 der Aussenkatheter 30 dieses verletzen kann.

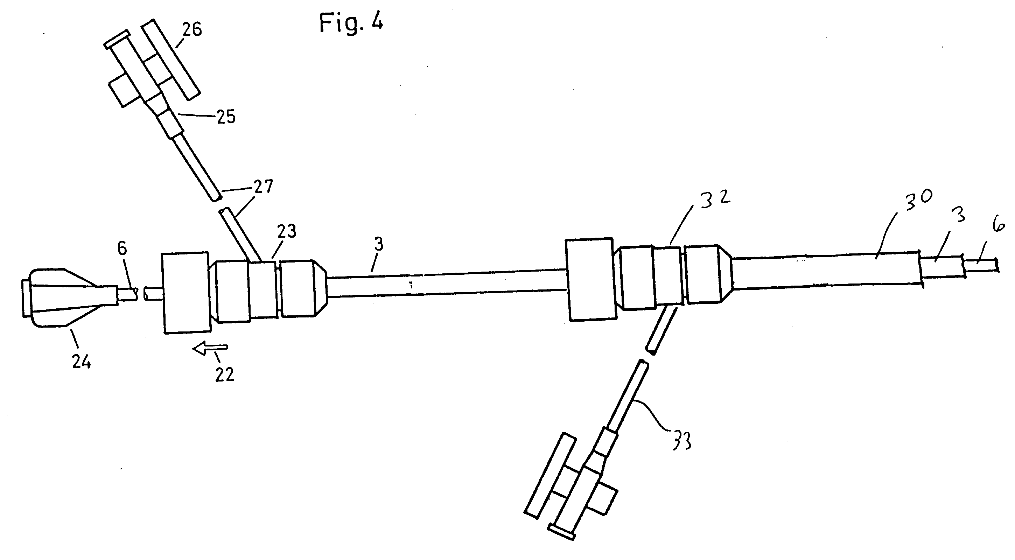

Der Innenkatheter 6 ist gemäss Fig. 4 am proximalen Ende mit einem an sich bekannten Verbindungsstück 24 versehen, das zum Einspritzen beispielsweise von Kontrastmittel sowie zum Einführen eines Führungsdrahtes dient. Der erste Aussenkatheter 3 ist an seinem proximalen Ende mit einem Anschluss- und Dichtungsstück 23 verbunden, das eine hier nicht gezeigte Dichtung aufweist, die verschiebbar am Innenkatheter 6 anliegt. Das Anschluss- und Dichtungsstück 23 ist in üblicher Weise mit einer Abzweigung 25 und einem Hahn 26 sowie einer flexiblen Schlauchleitung 27 versehen, wobei die Schlauchleitung mit dem Lumen 8 des ersten Aussenkatheters 3 verbunden ist. Der zweite Aussenkatheter 30 ist an seinem proximalen Ende ebenfalls mit einem Anschluss- und Dichtungsstück 32 verbunden, das gleich ausgebildet sein kann wie das Stück 23, jedoch in Anpassung an den grösseren Aussendurchmesser des Katheters 30 entsprechend grösser ausgebildet ist. Eine entsprechende Schlauchleitung 33 ist mit dem Lumen des zweiten Aussenkatheters 30 verbunden. Der erste Aussenkatheter 3 kann somit in Längsrichtung auf dem Innenkatheter 6 und der zweite Aussenkatheter 30 auf dem ersten Aussenkatheter 3 ebenfalls in Längsrichtung teleskopisch verschoben werden. Das Schlauchstück 30b des zweiten Aussenkatheters 30 ist über seine gesamte Länge auf dem ersten Aussenkatheter 3 gleitverschieblich geführt, die Festigkeitsanforderungen an den zweiten Aussenkatheter 30 sind somit vergleichsweise gering.

Die Verwendung der erfindungsgemässen Vorrichtung wird nachfolgend erläutert.

Zum Montieren der Endoprothese 1 wird diese auf den Innenkatheter 6 aufgeschoben, wobei die Aussenkatheter 3 und 30 wenigstens bis zu einer Fläche 16 zurückgezogen sind. Durch Vorschieben des ersten Aussenkatheters 3 über die Endoprothese 1 und anschliessend durch Vorschieben des zweiten Aussenkatheters 30 wird die Endoprothese 1 gefaltet und gespannt, bis sie schliesslich gemäss Fig. 1 in der Vorrichtung fixiert, und die Mündung 30b an der Schulter 5 der Spitze 7 anliegt.

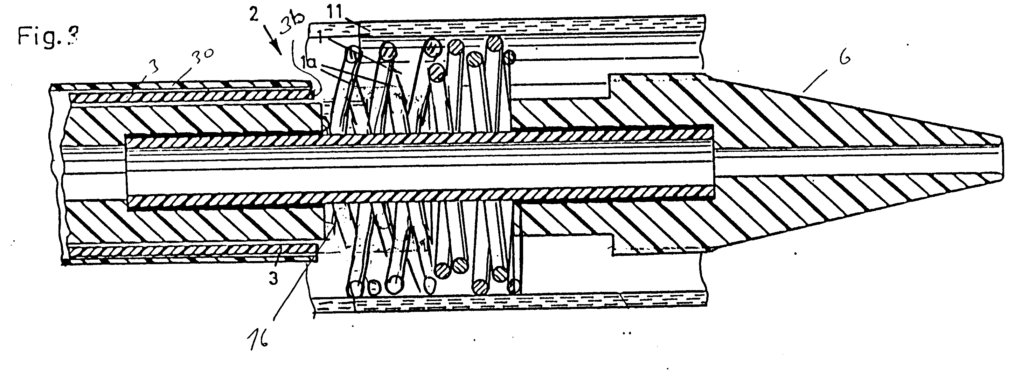

Die montierte Endoprothese 1 wird nun in an sich bekannter Weise mit der Vorrichtung 2 beispielsweise in das Gefäss 11 (Fig. 2) eingeführt. Zum Freisetzen der Endoprothese 1 wird der zweite Aussenkatheter 30 am Anschluss- und Dichtungsstück 32 zurückgezogen, bis er die in Fig. 2 gezeigte Lage erreicht. Die Endoprothese 1 wird dadurch im wesentlichen im Teilbereich B freigesetzt und kann in diesem Bereich expandieren. Bei der Anordnung gemäss Fig. 2 kann die Endoprothese 1 wieder vollständig komprimiert werden, indem der zweite Aussenkatheter 30 wieder vorgeschoben wird in die in Fig. 1 gezeigte Position. Dies ist möglich, da gemäss Fig. 2 die Endoprothese 1 in ihrem Bereich A vom ersten Aussenkatheter 3 durch Reibung festgehalten ist.

Um die Endoprothese vollständig freizusetzen, wird ausgehend von der Anordnung gemäss Fig. 2 nun auch der erste Aussenkatheter 3 zurückgezogen, bis eine Mündung hinter der Fläche 16 liegt. Die Endoprothese 1 ist nun gegen die Innenseite des Gefässes 11 gespannt und stützt dieses. Da die Endoprothese 1 nun erweitert ist, lässt sich die Vorrichtung 2 durch die Endoprothese 1 hindurch aus dem Gefäss 11 entfernen, wobei diese Endoprothese 1 im Gefäss 11 verbleibt.

Die beiden Schlauchstücke 3b und 30b sind vorzugsweise aus einem Kunststoff hergestellt, der vergleichsweise gute Gleiteigenschaften aufweist und der sich für diesen Zweck auch sonst gut eignet. Vorzugsweise ist dieser Kunststoff Polytetrafluoräthylen. Denkbar ist auch eine Ausführung, bei der mehr als zwei Aussenkatheter vorgesehen sind, die teleskopisch verschiebbar sind und jeweils anteilig die Endoprothese überspannen.

## Patentansprüche

1. Vorrichtung zum Freisetzen einer selbstexpandierenden Endoprothese (1), mit einem flexiblen länglichen Aussenkatheter (3) mit einem distalen Ende und einem proximalen Ende und mit einem koaxial zum Aussenkatheter (3) angeordneten flexiblen länglichen Innenkatheter (6), der an einem distalen Ende eine Spitze (7) und proximal dieser Mittel (14) zur Aufnahme der Endoprothese (1) aufweist, wobei zur Fixierung der Endoprothese (1) der Aussenkatheter (3) in seiner Längsrichtung über die Mittel (14) schiebbar und zu deren Freigabe zurückziehbar ist, dadurch gekennzeichnet, dass koaxial zum Aussenkatheter (3) an diesem ein zweiter, mit einer distalen Mündung (30b) an die Spitze (7) anlegbarer Aussenkatheter (30) angeordnet ist, der auf dem ersten Aussenkatheter (3) in Längsrichtung verschiebbar ist, wobei die genannte Spitze (7) die axiale Verschiebbarkeit des zweiten Aussenkatheters (30) relativ zum ersten Aussenkatheter (3) in distaler Richtung begrenzt, dass bei einer eingesetzten Endoprothese (1) der zweite Aussenkatheter (30) mit einem distalen Ende (30b) den ersten Aussenkatheter (3) distal überragt, derart, dass die Endoprothese (1) mit einem distalen Bereich (B) sich gegen eine Innenseite (31) des zweiten Aussenkatheters (30) und mit einem proximalen Bereich (A) gegen eine Innenseite des ersten Aussenkatheters (3) spannt und zur Freigabe der Endoprothese (1) der zweite Aussenkatheter (30) und der erste Aussenkatheter (3) auf dem Innenkatheter (6) von der Endoprothese (1) zurückziehbar sind.

2. Vorrichtung nach Anspruch 1, dadurch gekennzeichnet, dass die beiden Aussenkatheter (3,30) unabhängig voneinander von der Endoprothese (1) zurückziehbar sind.

3. Vorrichtung nach Anspruch 1 oder 2, dadurch gekennzeichnet, dass der zweite Aussenkatheter (30) ein rohrförmiges, flexibles Schlauchstück (30a) aufweist, an dem proximal ein Anschluss- und Dichtungsstück (32) befestigt ist, das den zweiten Aussenkatheter (30) gegen den ersten Aussenkatheter (3) abdichtet und auf diesem verschiebbar ist.

4. Vorrichtung nach einem der Ansprüche 1 bis 3, dadurch gekennzeichnet, dass der zweite Aussenkatheter (30) an seinem distalen Ende mit einer kreisförmigen Mündung (30b) endet.

5. Vorrichtung nach einem der Ansprüche 1 bis 4, dadurch gekennzeichnet, dass die Spitze (7) des Innenkatheters (6) eine Schulter (5) aufweist, welche den zweiten Aussenkatheter (30) an seinem distalen Ende seitlich überragt.

6. Vorrichtung nach einem der Ansprüche 1 bis 5, dadurch gekennzeichnet, dass der zweite Aussenkatheter (30) wenigstens an seinem distalen Ende eine glatte Innenseite aufweist.

7. Vorrichtung nach einem der Ansprüche 2 bis 6, dadurch gekennzeichnet, dass das Schlauchstück (30a) aus Polytetrafluoräthylen oder einem ähnlich gut gleitenden Kunststoff hergestellt ist.

8. Vorrichtung nach einem der Ansprüche 3 bis 7, dadurch gekennzeichnet, dass die Wandstärke des Schlauchstückes (30a) des zweiten Aussenkatheters (30) im wesentlichen der Wandstärke eines Schlauchstücks des ersten Aussenkatheters (3) entspricht.

9. Vorrichtung nach einem der Ansprüche 1 bis 8, dadurch gekennzeichnet, dass der Innenkatheter (6) auf seiner Aussenseite eine kreiszylindrische Ausnehmung (14) zur Aufnahme der Endoprothese (1) aufweist.

10. Vorrichtung nach einem der Ansprüche 1 bis 9, dadurch gekennzeichnet, dass der Innenkatheter (6) ein Zwischenstück (9) aufweist, welches die Spitze (7) mit einem distal angeordneten Rohrstück (10) verbindet und das einen Aussendurchmesser (F) aufweist, der kleiner ist als ein Aussendurchmesser (C) des Rohrstückes (10).

## Claims

1. Device for releasing a self-expanding endoprosthesis (1), comprising a flexible elongated outer catheter (3) having a distal end and a proximal end and comprising a flexible elongated inner catheter (6) arranged coaxially with respect to the outer catheter (3), said inner catheter having at a distal end a tip (7) and, proximal to the latter, means (14) for receiving the endoprosthesis (1), whereby the outer catheter (3) is capable of being pushed in its longitudinal direction via the means (14) in order to fix the endoprosthesis (1) and is capable of being retracted in order to release said endoprosthesis, characterised in that on the outer catheter (3) there is arranged, coaxially with respect to the latter, a second outer catheter (30) which is capable of being applied with a distal orifice (30b) onto the tip (7), said second outer catheter being displaceable on the first outer catheter (3) in the longitudinal direction, whereby the stated tip (7) limits the capacity of the second outer catheter (30) for axial displacement relative to the first outer catheter (3) in the distal direction, in that when an endoprosthesis (1) is inserted the second outer catheter (30) protrudes distally beyond the first outer catheter (3) with a distal end (30b) in such a manner that the endoprosthesis (1) is tightened with a distal region (B) against an inner side (31) of the second outer catheter (30) and with a proximal region (A) against an inner side of the first outer prosthesis (3) and in order to release the endoprosthesis (1) the second outer catheter (30) and the first outer catheter (3) on the inner catheter (6) are capable of being retracted from the endoprosthesis (1).

2. Device according to Claim 1, characterised in that the two outer catheters (3, 30) are capable of being retracted from the endoprosthesis (1) independently of one another.

3. Device according to Claim 1 or 2, characterised in that the second outer catheter (30) has a tubular, flexible length of hose (30a), on which a connecting and sealing piece (32) is secured proximally which seals the second outer catheter (30) in relation to the first outer catheter (3) and is displaceable on the latter.

4. Device according to one of Claims 1 to 3, characterised in that the second outer catheter (30) terminates at its distal end with a circular orifice (30b).

5. Device according to one of Claims 1 to 4, characterised in that the tip (7) of the inner catheter (6) has a shoulder (5) which protrudes laterally beyond the second outer catheter (30) at the distal end thereof.

6. Device according to one of Claims 1 to 5, characterised in that the second outer catheter (30) has a smooth inner side at least at its distal end.

7. Device according to one of Claims 2 to 6, characterised in that the length of hose (30a) is produced from polytetrafluoroethylene or a synthetic material that slides similarly well.

8. Device according to one of Claims 3 to 7, characterised in that the wall thickness of the length of hose (30a) pertaining to the second outer catheter (30) corresponds substantially to the wall thickness of a length of hose pertaining to the first outer catheter (3).

9. Device according to one of Claims 1 to 8, characterised in that the inner catheter (6) has on its outer side a circular cylindrical recess (14) for receiving the endoprosthesis (1).

10. Device according to one of Claims 1 to 9, characterised in that the inner catheter (6) has an intermediate piece (9) which connects the tip (7) to a distally arranged length of tubing (10) and which has an outside diameter (F) smaller than an outside diameter (C) of the length of tubing (10).

## Revendications

1. Dispositif pour libérer une endoprothèse auto-extensible (1), comportant un cathéter allongé flexible (3) possédant une extrémité distale et une extrémité proximale et un cathéter intérieur allongé flexible (6), qui est disposé coaxialement par rapport au cathéter extérieur (3) et possède, sur une extrémité distale, une pointe (7) et, sur le côté proximal de cette extrémité, des moyens (14) pour recevoir l'endoprothèse (1), auquel cas pour la fixation de l'endoprothèse (1), le cathéter extérieur (3) peut être repoussé dans sa direction longitudinale par-dessus les moyens (14) et peut être rétracté par-dessus ces moyens pour la libération de l'endoprothèse, caractérisé en ce que coaxialement au cathéter extérieur (3) est disposé, sur ce dernier, un second cathéter extérieur (30), qui peut être appliqué par une embouchure distale (30b) sur la pointe (7) et qui peut être déplacé dans la direction longitudinale sur le premier cathéter extérieur (3), ladite pointe (7) limitant la mobilité axiale du second cathéter extérieur (30) par rapport au premier cathéter extérieur (3) dans la direction distale de telle sorte que lorsqu'une endoprothèse (1) est insérée, le second cathéter extérieur (30) fait saillie distalement par une extrémité distale (30b) au-delà du premier cathéter extérieur (3) de sorte que l'endoprothèse (1) est serrée par une extrémité distale (B) contre une face intérieure (31) du second cathéter extérieur (30) et, par une extrémité proximale (A), contre une face intérieure d'un premier cathéter extérieur (3) et que pour la libération de l'endoprothèse (1), le second cathéter extérieur (30) et le premier cathéter extérieur (3) peuvent être rétractés sur le cathéter intérieur (6) à partir de l'endoprothèse (1).

2. Dispositif selon la revendication 1, caractérisé en ce que les deux cathéters extérieurs (3, 30) peuvent être rétractés de l'endoprothèse (1), indépendamment l'un l'autre.

3. Dispositif selon la revendication 1 ou 2, caractérisé en ce que le second cathéter extérieur (30) possède un élément de tuyau souple de forme tubulaire (30a), sur lequel est fixé, sur le côté proximal, un élément de raccordement et d'étanchéité (32), qui étanchéifie le second cathéter extérieur (30) par rapport au premier cathéter extérieur (3) et peut être déplacé sur ce dernier.

4. Dispositif selon l'une des revendications 1 à 3, caractérisé en ce que le second cathéter extérieur (30) se termine, au niveau de son extrémité distale, par une embouchure de forme circulaire (30b).

5. Dispositif selon l'une des revendications 1 à 4, caractérisé en ce que la pointe (7) du cathéter intérieur (6) comporte un épaulement (5), qui fait saillie latéralement hors du second cathéter extérieur (30), au niveau de son extrémité distale.

6. Dispositif selon l'une des revendications 1 à 5, caractérisé en ce que le second cathéter extérieur (30) comporte une face intérieure lisse au moins sur son extrémité distale.

7. Dispositif selon l'une des revendications 2 à 6, caractérisé en ce que l'élément de tuyau (30a) est réalisé en polytétrafluoroéthylène ou en une matière plastique ayant une propriété de glissement aussi bonne.

8. Dispositif selon l'une des revendications 3 à 7, caractérisé en ce que l'épaisseur de paroi de l'élément de tuyau (30a) du second cathéter extérieur (30) correspond essentiellement à l'épaisseur de paroi d'un élément de tuyau du premier cathéter extérieur (3).

9. Dispositif selon l'une des revendications 1 à 8, caractérisé en ce que le cathéter intérieur (6) comporte, sur sa face extérieure, un évidement en forme de cylindre circulaire (14) servant à recevoir l'endoprothèse (1).

10. Dispositif selon l'une des revendications 1 à 9, caractérisé en ce que le cathéter intérieur (6) possède un élément intermédiaire (9), qui relie la pointe (7) à un élément tubulaire (10) disposé de façon distale et possède un diamètre extérieur (F) qui est inférieur à un diamètre extérieur (C) de l'élément tubulaire (10).
